(19) Europäisches Patentamt
European Patent Office
Office européen des brevets

(11) Numéro de publication: **0 230 166**
**A1**

(12) # DEMANDE DE BREVET EUROPEEN

(21) Numéro de dépôt: **86402611.7**

(22) Date de dépôt: **25.11.86**

(51) Int. Cl.⁴: **G 01 N 33/543**, G 01 N 33/569

(30) Priorité: **25.11.85 FR 8517377**

(43) Date de publication de la demande: **29.07.87**
**Bulletin 87/31**

(84) Etats contractants désignés: **AT BE CH DE ES FR GB GR IT LI LU NL SE**

(71) Demandeur: **INSTITUT NATIONAL DE LA SANTE ET DE LA RECHERCHE MEDICALE (INSERM), 101, rue de Tolbiac, F-75654 Paris Cedex 13 (FR)**

(72) Inventeur: **Quash, Gérard A., Village de l'Ouest 12 Allée des Charmilles, F-69340 Francheville (FR)**

(74) Mandataire: **Ores, Irène et al, CABINET ORES 6, Avenue de Messine, F-75008 Paris (FR)**

(54) **Nouveaux réactifs de diagnostic.**

(57) La présente invention est relative à de nouveaux réactifs de diagnostic.

Le réactif comprend un support solide qui porte des chaînes latérales constituées par un dérivé d'hydrazine et/ou un acide aminé, auxquelles sont fixées par liaison chimique des molécules d'antigène ou d'anticorps. Procédé de production d'un tel réactif.

Application à la détection d'anticorps ou d'antigènes dans un fluide biologique.

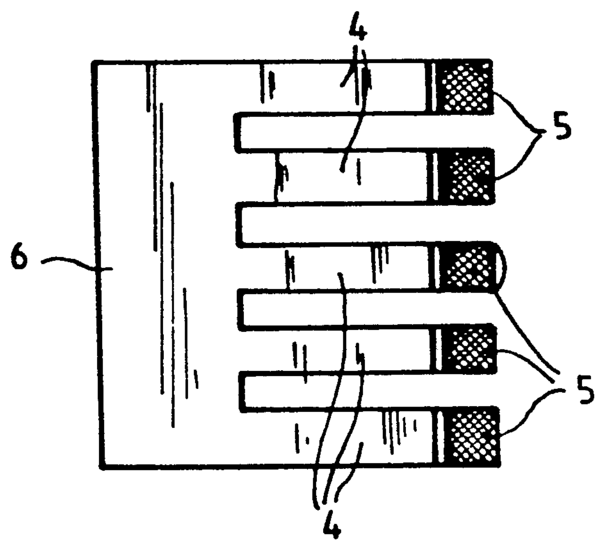


EP 0 230 166 A1

La présente invention est relative à de nouveaux réactifs de diagnostic, destinés, en particulier, à l'application en virologie.

La technique ELISA (Enzyme linked immunosorbent assay) est une méthode immuno-enzymatique, d'une très grande sensibilité, de titrage d'anticorps, qui consiste à utiliser un complexe immunoadsorbant constitué par un antigène fixé sur un support solide, pour capter les anticorps spécifiques contenus dans le milieu biologique, tel que sérum, à tester, l'immun-complexe ainsi obtenu étant détecté par un anticorps anti-espèce marqué par une enzyme, après quoi on ajoute un substrat spécifique de l'enzyme, dont la dégradation par l'enzyme fait apparaître un produit coloré ; l'intensité de la coloration est proportionnelle à la quantité d'enzyme qui réagit et donc à la quantité d'anticorps présente dans le milieu biologique testé ; l'intensité de la coloration peut être appréciée à l'oeil nu ou mesurée par photométrie.

Dans ce type de méthode immuno-enzymatique utilisant une phase hétérogène, la nature de cette phase solide qui sert de support à la réaction immunologique est très importante car elle détermine la sensibilité de la mesure.

Les supports qui ont été proposés pour le test ELISA sont, notamment, des grains de polyacrylamide activés par le glutaraldéhyde (AVRAMEAS et TERNYNCK, Immunochemistry, 1969, 6, p 53-65) ; des particules de cellulose activée par le bromure de cyanogène, sur lesquelles des anticorps sont fixés par liaisons covalentes (ENGVALL et PERLMANN, Immunochemistry, 1971, 8, 871-874) ; des tubes en polystyrène sur la surface desquels des antigènes sont fixés par simple adsorption physique (ENGVALL et PERLMANN, J. Immun. 1972, 160, 129-136) ; des disques de papier activé par le bromure de cyanogène et sur lesquels est fixé un anticorps ou un antigène (BRIGHTON et Coll. Scand. 1974, 29, 166-174). Cependant, les supports les plus répandus actuellement sont les surfaces en matière plastique qui se présentent sous forme

de microplaques de 96 puits à fond plat, en U ou en V, en polystyrène ou en PVC, ou de barrettes de 8 puits. Cependant les antigènes ou les anticorps sont fixés sur ces microplaques ou analogues par simple adsorption physique.

Lors de l'adsorption physique des protéines, ces dernières subissent un changement de structure qui dépend à la fois de la nature de la protéine et de la composition de la surface du support insoluble. Une conséquence de ce changement de structure peut se traduire par une perte d'antigénicité de la protéine donnée. Dans le cas des antigènes viraux, par exemple, le virus cytomégalique et le virus de la dengue, il a été noté que certains anticorps monoclonaux peuvent neutraliser le virus ou réagir avec le virus dans les cellules infectées, mais ne donnent pas de réaction avec le virus adsorbé sur des surfaces en matière plastique. Cette difficulté pourrait être levée par le couplage covalent de l'antigène viral par l'intermédiaire soit de ses résidus glucidiques, soit de ses résidus acides aminés, à des supports insolubles. Comme décrit précédemment, les supports les plus répandus sont les plaques de microtitration de 96 puits ou les barrettes de 8 puits. La lecture de la réaction ELISA avec ce type de matériau nécessite un appareillage relativement coûteux. De ce fait, il est mal adapté (1) aux enquêtes épidémiologiques sur le terrain, (2) aux tests de diagnostic effectués en dehors des grands centres, et (3) aux problèmes de sérodiagnostic dans des pays à infrastructure socio-économique faible.

La présente invention s'est en conséquence donné pour but de pourvoir à de nouveaux réactifs de diagnostic applicables notamment en virologie, qui répondent mieux aux nécessités de la pratique que les réactifs de diagnostic visant au même but, précédemment proposés, notamment en ce qu'ils préservent une intégrité fonctionnelle de l'antigène ou de l'anticorps fixé de manière covalente, supérieure à celle des réactifs de l'Art antérieur, en ce que leur mise en

oeuvre est facile et ne nécessite pas d'appareillage particulier et en ce que les réactifs de diagnostic conformes à la présente invention ne posent aucun problème de conservation dans le temps.

La présente invention a pour objet un nouveau réactif de diagnostic, caractérisé en ce qu'il comprend essentiellement un support solide pris dans le groupe qui englobe notamment les microplaques en polystyrène ou en PVC et les bandelettes, lequel support porte des chaînes latérales constituées par un dérivé d'hydrazine et/ou un acide aminé, auxquelles sont fixées par liaison chimique, des molécules d'antigène ou d'anticorps.

Selon un mode de réalisation préféré du réactif de diagnostic conforme à la présente invention, le support solide est constitué par une bandelette qui se compose d'une couche de matière textile appropriée fixée à une couche de matière thermoplastique telle que notamment le PVC ou le polystyrène.

Selon une disposition avantageuse de ce mode de réalisation, la couche en matière textile qu'il comprend est choisie parmi les fibres de polyamide (et notamment de "Nylon"), les matériaux à base de cellulose tels, notamment, que la cellulose, la nitrocellulose, l'acétate de cellulose, les polyesters et analogues.

Selon un autre mode de réalisation préféré du réactif de diagnostic conforme à la présente invention, les chaînes latérales fixées au support solide sont constituées par un composé pris dans le groupe qui comprend des dérivés d'hydrazine tels que les acides-hydrazides et la phénylhydrazine et les acides aminés, notamment les acides aminés contenant un groupement -SH.

Selon encore un autre mode de réalisation préféré du réactif de diagnostic conforme à la présente invention, les molécules fixées par liaison chimique aux chaînes latérales portées par le support solide, sont prises dans le groupe

qui comprend les antigènes viraux, les antigènes bactériens, les haptènes, les anticorps, les protéines plasmatiques, les acides ribonucléiques et désoxyribonucléiques, les immunoglobulines utilisables selon le cas comme antigènes ou anticorps.

Selon un mode de réalisation préféré du réactif de diagnostic conforme à la présente invention, les antigènes ou anticorps fixés par liaison chimique aux chaînes latérales portées par le support solide sont constitués par des protéines ou des glycoprotéines non oxydées et lesdites chaînes latérales sont constituées par un acide-hydrazide diazoté ou par un composé organique comportant un groupe carboxyle terminal.

Selon un autre mode de réalisation préféré du réactif de diagnostic conforme à la présente invention, les antigènes ou anticorps fixés par liaison chimique aux chaînes latérales portées par le support solide sont constitués par des glycoprotéines oxydées et lesdites chaînes latérales sont constituées par un composé pris dans le groupe qui comprend des dérivés d'hydrazine tels que les acides-hydrazides et la phénylhydrazine, et les acides aminés, notamment les acides aminés contenant un groupe -SH, tels que la cystéine.

Selon un mode de réalisation préféré du réactif de diagnostic conforme à la présente invention, celui-ci comprend un antigène du virus cytomégalique (CMV), préalablement oxydé et fixé par couplage chimique par ses résidus glucidiques aux chaînes latérales constituées par un dérivé d'hydrazide, portées par le support solide tel que microplaque en PVC ou en polystyrène ou bandelette en fibres de matière textile elle-même fixée sur un support en matière thermoplastique, telle que PVC ou polystyrène notamment.

Selon encore un autre mode de réalisation préféré du réactif de diagnostic conforme à la présente invention, dans le cas où le support solide est une bandelette conforme à la présente invention, la couche de matière textile est

avantageusement fixée sur la couche de matière thermoplastique inerte, par soudage, et de préférence par soudage haute fréquence.

Selon encore un autre mode de réalisation préféré du réactif de diagnostic conforme à la présente invention, la couche de matière textile est en matériau à base de cellulose, oxydé.

Selon un mode de réalisation préféré du réactif de diagnostic conforme à la présente invention, la couche textile est fixée à une partie seulement de la couche de matière thermoplastique et le réactif de diagnostic présente sensiblement la forme d'une bandelette.

Selon une disposition avantageuse de ce mode de réalisation, la surface de la partie de la couche de matière thermoplastique à laquelle est fixée la couche de matière textile à laquelle sont fixés des antigènes ou des anticorps par l'intermédiaire de chaînes latérales, est comprise entre 1/5 et 1/50 environ de la surface totale de la couche de matière thermoplastique.

Selon une autre disposition avantageuse de ce mode de réalisation, une pluralité de bandelettes sont associées entre elles par l'intermédiaire d'une bande de support commune.

Selon une modalité avantageuse de cette disposition, les bandelettes présentent une ligne d'affaiblissement à la jonction de chacune d'entre elles à la bande de support commune.

Selon un mode de réalisation avantageux du réactif de diagnostic conforme à l'invention, lorsque la couche de matière thermoplastique ou le support solide est en polystyrène, celui-ci est traité au préalable pour le convertir en polyaminostyrène.

La présente invention a également pour objet un procédé de production du nouveau réactif conforme à l'invention, qui est caractérisé en ce que le support solide qu'il

comprend comporte des groupes amine primaire libres, en ce que ledit support est substitué par des chaînes latérales par couplage de celles-ci avec lesdits groupes $NH_2$ libres et en ce que des molécules d'un antigène ou d'un anticorps approprié sont fixées par liaisons de covalence aux extrémités libres des chaînes latérales préalablement fixées par couplage sur le support solide.

Selon un mode de réalisation du procédé de production dudit réactif, lorsque le support solide est constitué par une bandelette dans laquelle une couche de matière textile est associée à une couche de matière thermoplastique, ledit procédé comprend :

- une première étape au cours de laquelle une bandelette faite de fibres d'une matière textile est substituée par des chaînes latérales couplées aux groupements $NH_2$ libres des fibres de matière textile ;
- une deuxième étape au cours de laquelle des molécules d'un antigène ou d'un anticorps convenable sont fixées par liaisons de covalence aux extrémités libres des chaînes latérales fixées à la bandelette au cours de la première étape,
- la bandelette de fibres de matière textile étant fixée à la couche de matière thermoplastique soit préalablement à la fixation des chaînes latérales et de l'antigène ou de l'anticorps anti-espèce, soit postérieurement à ces étapes de traitement.

Selon un mode de mise en oeuvre préféré du procédé de production du réactif de diagnostic conforme à la présente invention, le support comportant des groupes $NH_2$ libres, est substitué par des chaînes latérales d'un dérivé d'hydrazine, par succinylation desdits groupes $NH_2$ par de l'anhydride succinique, suivie de la transformation de la fonction acide carboxylique terminale en hydrazide, par réaction avec l'hydrazine, en présence d'un carbodiimide approprié.

Selon un autre mode de mise en oeuvre préféré du procédé de production du réactif de diagnostic conforme à la

présente invention, le support comportant des groupes $NH_2$ libres, est substitué par des chaînes latérales d'un acide aminé comportant un groupe -SH, notamment par la cystéine, en présence d'un carbodiimide approprié.

Selon un mode de mise en oeuvre approprié du procédé de production du réactif de diagnostic conforme à la présente invention, l'antigène ou l'anticorps comporte un résidu glucidique comprenant au moins un groupe $-CH_2OH$ qui est oxydé en un groupe -CHO préalablement à la fixation de l'antigène ou de l'anticorps à une chaîne latérale du support solide.

Selon une modalité avantageuse de ce mode de mise en oeuvre, l'antigène ou l'anticorps est fixé au support, par réaction chimique entre son groupe -CHO terminal et le groupe amine primaire libre terminal porté par la chaîne latérale du support solide.

Selon un mode de mise en oeuvre préféré du procédé de production du réactif de diagnostic conforme à l'invention, celui-ci comprend l'association d'une bandelette de matière textile à une couche de matière thermoplastique.

Selon une disposition avantageuse de ce mode de mise en oeuvre, la bandelette de matière textile est en fibres de polyamides.

Selon une autre disposition avantageuse de ce mode de mise en oeuvre, la bandelette de matière textile est en fibres de polyester.

Selon encore une autre disposition avantageuse de ce mode de mise en oeuvre, la bandelette de matière textile est une bandelette en fibres de matériau à base de cellulose pris dans le groupe de la cellulose, de la nitrocellulose et de l'acétate de cellulose.

Selon une modalité avantageuse de cette disposition, la bandelette en fibres de matériau à base de cellulose est soumise à un processus d'oxydation préalablement à la fixation des chaînes latérales de dérivé d'hydrazine.

Selon une autre disposition avantageuse de ce mode

de mise en oeuvre, après la fixation de chaînes latérales de dérivé d'hydrazine sur les bandelettes de fibres de matière textile oxydées, les groupes carbonyle libres qui n'ont pas réagi au cours de l'étape de fixation des chaînes latérales, avec le dérivé d'hydrazine, sont réduits en alcools par un agent réducteur approprié tel que, notamment, le borohydrure de sodium.

Selon un autre mode de mise en oeuvre du procédé de production du réactif de diagnostic conforme à l'invention, l'antigène ou l'anticorps est fixé au support solide par réaction chimique entre son groupe-$NH_2$ et le groupe acide-hydrazide de la chaîne latérale fixée audit support.

Selon une disposition avantageuse de ce mode de mise en oeuvre, l'antigène ou l'anticorps fixé par couplage chimique est pris dans le groupe des protéines, des holoprotéines, des glycoprotéines.

Selon encore une autre disposition avantageuse de ce mode de mise en oeuvre, les chaînes latérales du support solide, constituées par un acide-hydrazide, sont diazotées en un acide-azide auquel sont couplés par réaction chimique des antigènes ou des anticorps constitués par des protéines, des holoprotéines ou des glycoprotéines non oxydées, ainsi que par des acides ribo- et désoxyribo-nucléiques.

La présente invention a, en outre, pour objet un procédé de détection d'anticorps ou d'antigènes dans un fluide biologique tel que sérum, qui comprend :
- une première étape au cours de laquelle un réactif de diagnostic conforme à la présente invention, est immergé pendant un temps approprié dans une solution de sérum ;
- une deuxième étape au cours de laquelle le réactif retiré de la solution de sérum, puis lavé, est introduit dans une solution contenant des anticorps anti-espèce à détecter, marqués par une enzyme puis est lavé à plusieurs reprises à l'aide d'un tampon de composition convenable ;
- une troisième étape au cours de laquelle le réactif de diagnostic est introduit dans une solution contenant un substrat avantageusement associé à un accepteur d'électrons, qui précipite sur le réactif en présence d'anticorps ou d'antigènes spécifiques, en donnant audit réactif une coloration pourpre plus ou moins intense, selon la quantité de produit formé, laquelle coloration, par comparaison à une échelle de colorations standard, mesure la quantité d'anticorps ou d'antigènes présente dans le fluide biologique testé.

Selon un mode de mise en oeuvre avantageux de ce procédé de détection, les molécules fixées par couplage chimique aux chaînes latérales constituées par un dérivé d'hydrazine, du réactif de diagnostic, sont des molécules d'immunoglobulines (IgG, IgM, IgE ...), préalablement oxydées, couplées par réaction chimique de la fonction $-NH_2$ libre d'une chaîne latérale de dérivé d'hydrazine fixée au support du réactif de diagnostic, avec la fonction -CHO libre de la molécule d'immunoglobuline-CHO correspondante, avec formation d'hydrazone.

Selon un autre mode de mise en oeuvre avantageux de ce procédé de détection, les anticorps dirigés contre l'antigène à détecter sont marqués directement par une enzyme et sont introduits dans le fluide biologique à tester, dans une dilution d'un tampon approprié contenant un détergent non-ionique, du borate de sodium, un acide aminé approprié et du chlorure de sodium, ajusté à pH 8,1 environ.

Si l'antigène est présent dans le fluide biologique, la quantité d'anticorps marqué à l'enzyme restant disponible est moindre et, de ce fait, la réaction avec l'antigène fixé sur le support solide est diminuée, ce qui indique la présence de l'antigène dans le fluide biologique.

Selon un autre mode de mise en oeuvre avantageux de ce procédé de détection, les molécules fixées par couplage chimique aux chaînes latérales constituées par un dérivé d'hydrazide, du support solide, sont des antigènes viraux, notamment des antigènes du virus cytomégalique (CMV), préalablement oxydés.

Selon une disposition avantageuse de ce mode de mise en oeuvre, le réactif de diagnostic comprenant un antigène du virus cytomégalique (CMV) est mis en oeuvre sous forme de plaques ou de bandelettes, pour détecter de façon spécifique des anticorps anti-CMV neutralisants présents dans un sérum à tester, par mise en contact de la bandelette ou de la plaque sur laquelle sont couplés des antigènes CMV, avec le sérum à tester, suivie d'un lavage par un tampon approprié et d'une mise en contact avec des anticorps anti-IgG humaines marqués par une enzyme ou par la biotine.

Selon une autre disposition avantageuse de ce mode de mise en oeuvre, le réactif de diagnostic comprenant un antigène du virus cytomégalique (CMV) est mis en oeuvre sous forme de plaque pour détecter des anticorps anti-CMV neutralisants et notamment des IgG anti-CMV dans un sérum à tester, en répartissant ledit sérum, dilué à l'aide d'un tampon approprié, suivant des dilutions appropriées, dans les puits de la plaque sur laquelle sont fixés par couplage d'une part l'antigène CMV et d'autre part de l'antigène de contrôle préparés respectivement à partir d'homogénéisats de cellules infectées par le CMV et à partir d'homogénéisats de cellules non-infectées, après quoi sont introduites dans chaque puits des quantités identiques d'une solution de sérum, de chèvre par exemple, contenant des anticorps anti-IgG humaines par exemple, marqués à la phosphatase alcaline, et l'activité enzymatique est révélée, après un temps de contact suffisant à 37°C environ, à l'aide d'un substrat approprié, tel que, notamment, le substrat p-nitrophénylphosphate.

Selon encore un autre mode de mise en oeuvre avantageux de ce procédé de détection, les molécules fixées par couplage chimique aux chaînes latérales constituées par un dérivé d'hydrazide, du support solide, sont des antigènes bactériens, tels que, notamment, la protéine A provenant de Staphylococcus aureus, couplée chimiquement aux chaînes latérales diazotées du support du réactif de diagnostic.

Selon un autre mode de mise en oeuvre avantageux de ce procédé de détection, les molécules fixées par couplage chimique aux chaînes latérales constituées par un dérivé d'hydrazide, du support du réactif de diagnostic, sont un haptène, notamment un produit de couplage caséine-spermine couplé chimiquement aux chaînes latérales diazotées de la bandelette en fibres de matière textile.

Selon encore un autre mode de mise en oeuvre avantageux de ce procédé de détection, les molécules fixées par couplage chimique aux chaînes latérales constituées par un dérivé d'hydrazide, du support du réactif de diagnostic, sont un acide désoxyribonucléique couplé chimiquement aux chaînes latérales diazotées de la bandelette de support.

Selon un autre mode de mise en oeuvre avantageux de ce procédé de détection, les molécules fixées par couplage chimique aux chaînes latérales constituées par un dérivé d'hydrazide, du support du réactif de diagnostic, sont des anticorps pris, entre autres, dans le groupe qui comprend les anticorps anti-Salmonella préalablement oxydés.

Selon encore un autre mode de mise en oeuvre avantageux de ce procédé de détection, le tampon utilisé pour le lavage des bandelettes ou des plaques de réactif de diagnostic à leur sortie du fluide biologique, est le même que celui utilisé pour la dilution des anticorps anti-espèce marqués par une enzyme.

Selon un autre mode de mise en oeuvre du procédé de détection conforme à l'invention, le substrat est constitué soit par une solution de peroxyde d'hydrogène associé à l' $\alpha$ -chloronaphtol ou à l'o-dianisidine ou encore à l'o-phénylènediamine, soit par une solution de p-nitrophénylphosphate, notamment.

Selon encore un autre mode de mise en oeuvre du procédé de détection conforme à la présente invention, les réactifs de diagnostic conformes à la présente invention sont saturés, préalablement à leur mise en oeuvre dans ledit procédé de détection, par des protéines prises, notamment, dans le groupe qui comprend la sérum-albumine bovine, les IgG de veau, la gélatine, dans le but d'éliminer l'adsorption non spécifique de protéines sériques pendant le procédé de détection par la réaction ELISA.

La présente invention a également pour objet un coffret, ou kit, de diagnostic prêt à l'emploi pour la réalisation du procédé de détection conforme aux dispositions qui précèdent, caractérisé en ce qu'il comprend, en combinaison :

- un réactif de diagnostic tel que défini dans ce qui précède, portant des antigènes ou anticorps appropriés ;
- une quantité appropriée d'anticorps marqués par une enzyme, dirigés contre l'antigène du réactif de diagnostic ;
- une quantité appropriée de tampon I ;
- une quantité appropriée de tampon II ;
- une pluralité de plaques de microtitration ;
- une quantité appropriée de substrat de l'enzyme ;
- une quantité appropriée d'accepteur d'électrons.

Selon un mode de réalisation préféré du coffret de diagnostic conforme à la présente invention, le réactif de diagnostic portant des antigènes ou des anticorps appropriés, est fixé par couplage chimique aux chaînes latérales constituées par un dérivé d'hydrazide portées par des plaques de microtitration et plus spécifiquement fixées dans chacun des puits desdites plaques.

Selon un autre mode de réalisation préféré du kit de diagnostic conforme à la présente invention, le réactif de diagnostic portant des antigènes ou des anticorps appropriés, est fixé par couplage chimique aux chaînes latérales portées par des bandelettes de support appropriées.

Pour la mise en oeuvre de la présente invention, on procède, de préférence, comme suit :

1. Le support solide se compose :
   - soit d'une microplaque comportant un nombre de puits convenable (microplaque de 96 puits ou barrette de 8 puits, par exemple) qui peut être en PVC ou de préférence en polyaminostyrène ;

- soit :
  . d'une bande de "Nylon" aminé (Zeta Probe) de 4 mm x 8 mm, par exemple, soudée sur un support en matière plastique inerte telle que PVC ou polystyrène par exemple, de 60 mm x 4 mm, par exemple. Le "Nylon" aminé contient 14 nmoles équivalent d'éthanolamine par $mm^2$ ;
  . ou bien d'une bande réalisée en une matière cellulosique.

2. La substitution des chaînes latérales sur le support a lieu selon le schéma suivant, qui a été décrit dans le Brevet Français INSERM 2 450 877 :

a) succinylation à l'aide d'anhydride succinique :

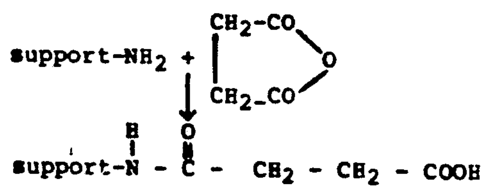

b) Conversion du -COOH terminal en acide-hydrazide, à l'aide d'hydrazine, en présence d'un carbodiimide hydrosoluble :

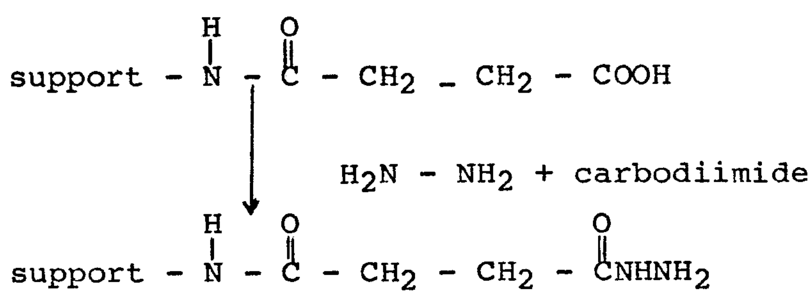


Après substitution, on trouve 7 nmoles équivalent d'acide-hydrazide par $mm^2$.

La terminaison acide-hydrazide (A.H.) permet de former :

- les hydrazones avec les résidus glucidiques des glycoprotéines oxydées,
- les liaisons peptidiques avec les $NH_2$ des protéines, holoprotéines ou des glycoprotéines non oxydées.

c) Oxydation de la fonction $CH_2OH$ de l'anticorps anti-espèce à fixer sur la chaîne latérale du support solide :
l'oxydation de la fonction $-CH_2OH$ en fonction -CHO est réalisée de préférence conformément aux modalités du Brevet Français INSERM 2 331 567.

d) Fixation de l'anticorps oxydé par réaction de sa fonction -CHO avec la fonction $-NH_2$ libre de la chaîne latérale acide-hydrazide du support solide.

3. Dans le cas où le support solide n'est pas un polyamide ("Nylon") mais une matière cellulosique, il est nécessaire de soumettre cette matière à un processus d'oxydation. Cette oxydation est effectuée, de préfé rence mais non limitativement, à l'aide de periodate de sodium en solution dans un tampon phosphate comme décrit dans le Brevet Américain CUATRECASAS & Al. N° 3 947 352.
L'efficacité de l'oxydation est contrôlée par le test d'aldéhyde en utilisant la N-méthyl benzothiazolone-hydrazone en présence de $FeCl_3$ comme décrit dans BACHRACH et RECHES, Anal. Biochem. 1966, <u>17</u>, 38-48.
Les bandelettes oxydées prennent une coloration bleu-vert foncé, tandis que les bandelettes contrôles non oxydées ne sont que légèrement teintées.
La densité optique (D.O.) à 630 nm de la bandelette oxydée et colorée par rapport à la D.O. de la bandelette non oxydée est de 0,400. Cette valeur correspond à 8 nmoles équivalents de glycéraldéhyde par $mm^2$.

4. En variante, dans le cas où le support solide est en une matière cellulosique, les étapes de succinylation et de conversion en acide-hydrazide décrites en 2) plus haut peuvent être précédées par une étape qui consiste à faire réagir de l'héxaméthylènediamine sur la bandelette ou plaque oxydée, suivie d'une réduction afin de stabiliser la base de Schiff formée entre l'amine primaire de l'hexaméthylènediamine et le carbonyle formé sur la cellulose oxydée.

5. Dans le cas où les chaînes latérales sont des dihydrazides-acides, par exemple de l'acide adipique-dihydrazide (ADH) de formule :

$$H_2N\text{-}HN\text{-}OC(CH_2)_4\text{-}CO\text{-}NH\text{-}NH_2$$

la réaction entre l'ADH et le carbonyle a lieu directement dans les conditions décrites, c'est-à-dire en solution à une concentration de l'ordre de 0,1 M, à un pH acide aux alentours de pH 2.

La vérification du nombre de groupements d'acide-hydrazide (AH) fixés est effectuée à l'aide d'acide trinitro-benzènesulfonique 0,001 M en solution dans du borate saturé.

La densité optique à 420 nm de la plaque ou bandelette AH préparée comme il vient d'être décrit, par rapport à la D.O. de la plaque ou bandelette oxydée mais non substituée par ADH, est de 0,300. Cette valeur correspond à 9 nmoles équivalent d'hydrazide/$mm^2$.

6. Une autre variante du schéma décrit en 2) ci-dessus consiste à coupler des antigènes et des anticorps non oxydés, sur les chaînes latérales portées par la couche de matière textile telle que polyamide, polyester ou dérivés cellulosiques ou portées par le support solide.

En pareil cas, les étapes c) et d) sont remplacées par une étape au cours de laquelle les groupes acide-hydrazide terminaux des chaînes latérales, qui répondent à la formule : $R\text{-}CONHNH_2$

sont diazotés en acide-azide de formule :

R-$CON_3$

auquel les antigènes ou les anticorps sont couplés chimiquement en mettant en oeuvre la technique décrite dans le Brevet Français INSERM N° 2 450 877.

7. La plaque ou bandelette de diagnostic obtenue est utilisée pour la réalisation de tests de détection d'anticorps suivant la technique ELISA, dans des fluides biologiques tels que sérums.

Les figures 1 et 2 des dessins annexés représentent :
- la figure 1 un mode de réalisation d'une bandelette à usage de réactif de diagnostic de réactions immuno-enzymatiques in situ,
- la figure 2 un mode de réalisation d'une bandelette multiple.

La bandelette représentée à la figure 1 se compose d'un support inactif 1 en PVC auquel est fixée par une ligne de soudure 3 une couche 2 de "Nylon" aminé substitué comme décrit plus haut.

Pour réaliser la fixation par soudure il est opportun d'utiliser les techniques de soudure haute fréquence.

La bandelette multiple représentée à la figure 2 se compose d'une pluralité de bandelettes 4 qui présentent chacune une structure semblable à celle de la bandelette représentée à la figure 1, réunies entre elles à leur extrémité opposée à celle qui porte la partie réactive 5, par un barreau 6 commun dont les bandelettes peuvent éventuellement être détachées en les rendant sécables, encore que dans le cas où la plaque à bandelettes multiples représentée à la figure 2 est réalisée de manière à être compatible avec une plaque de microtitration utilisée comme réservoir de réactif, les bandelettes sont introduites simultanément chacune dans le puits qui lui correspond, en sorte qu'il n'est pas nécessaire qu'elles soient sécables.

Il est avantageux de réaliser les extrémités libres des bandelettes de manière à leur donner une forme semblable à la forme du fond des cuvettes des plaques de microtitration en conjonction avec lesquelles les plaques à bandelettes multiples sont destinées à être utilisées.

Outre les dispositions qui précèdent, l'invention comprend encore d'autres dispositions, qui ressortiront de la description qui va suivre.

L'invention sera mieux comprise à l'aide du complément de description qui va suivre, qui se réfère à des exemples de préparation et d'application du réactif de diagnostic conforme à la présente invention.

Il doit être bien entendu, toutefois, que ces exemples sont donnés uniquement à titre d'illustration de l'objet de l'invention, dont ils ne constituent en aucune manière une limitation.

A. PREPARATION DE BANDELETTES-ANTIGENES

EXEMPLE 1 : préparation d'une bandelette couplée au virus cytomégalique (CMV)

a) Préparation de l'antigène CMV (AgCMV) :

AgCMV (expérimental) a été préparé à partir d'homogénéisats de cellules (fibroblastes) d'embryons humains $MRC_5$ infectées par le CMV pendant 6 à 8 jours.

$AgMRC_5$ (contrôle) a été préparé à partir d'homogénéisats de cellules $MRC_5$ non-infectées.

b) couplage des antigènes :

1 mg de protéine de chacun des deux antigènes a été préalablement oxydé conformément aux modalités décrites dans le Brevet Français INSERM 2 331 567.

Les antigènes ainsi oxydés ont été mis en contact avec les bandelettes-AH à raison de 20 µg/bandelette.

Pour l'AgCMV et l'$AgMRC_5$ la quantité de protéine fixée par bandelette (4 mm x 8 mm) était de 0,5 µg.

EXEMPLE 2 : Préparation d'une bandelette de diagnostic AH-CHO-IgG (protéine antigénique sérique)

1. A une bandelette de "Nylon" aminé (Zeta Probe) de 4 x 8 mm, contenant 14 nmoles amine équivalent d'éthanolamine par $mm^2$, soudée sur un support en PVC de 60 x 4 mm, est additionnée 1 mmole d'anhydride succinique solide ; après une nuit de contact en présence de tampon borate 0,1 M, pH 9,0, on met en contact avec 2 ml d'une solution d'hydrazine 0,2 M, pH 7,5 et de l'éthyl-3-3-diméthylaminopropylcarbodiimide (EDC) à l'état solide 0,05 M final. La préparation est incubée une nuit à + 4°C sur agitateur oscillant.

   Après substitution, on obtient 7 nmoles d'équivalent hydrazine par $mm^2$.

2. On fixe des immunoglobulines (IgG, IgM ou IgE) préalablement oxydées conformément aux modalités décrites dans le Brevet Français INSERM 2 331 567, par leur fonction -CHO à la fonction $-NH_2$ de la chaîne latérale acide-hydrazide.

Comme le montre le Tableau 1 ci-dessous, la quantité d'IgG fixée sur les bandelettes AH, est 20 fois supérieure à l'adsorption non spécifique sur les bandelettes COOH ;

TABLEAU 1

| Bandelette substituée à terminaison : | µg d'IgG oxydées fixés |
|---|---|
| -A.H.<br>-COOH | 4,5<br>0,23 |

la quantité d'IgG fixée a pu être déterminée avec précision en utilisant des IgG marquées par le N-succinimidyl-(2,3-$^3$H)propionate.

Il a été établi que la capacité de fixation maximale des IgG oxydées sur la bandelette, est de 40 µg d'IgG-CHO/bandelette, ce chiffre ayant été établi par représentation de Lineweaver-Burk à partir des chiffres réunis dans le Tableau 2 ci-après :

TABLEAU 2

Capacité de liaison des IgG oxydées sur une bandelette de "Nylon" aminé substituée par des chaînes latérales AH.

| µg/ml d'IgG*-CHO mises en contact avec la bandelette | 0 | 2 | 5 | 10 | 20 | 40 | 80 |
|---|---|---|---|---|---|---|---|
| µg d'IgG fixées par bandelette -AH | 0 | 0,17 | 0,62 | 1,70 | 3,66 | 6,30 | 11,61 |

IgG* : IgG marquées par le N-succinimidyl(2,3-$^3$H)-propionate

EXEMPLE 3 : préparation d'une bandelette de diagnostic dont l'antigène est une protéine bactérienne : la protéine A provenant de Staphylococcus aureus

Le couplage de la protéine A (I.B.F.) a été effectué sur les bandelettes diazotées selon la technique décrite plus haut, en procédant comme suit :

400 µg de protéine A ont été mis en contact avec 10 bandelettes diazotées.

La quantité de protéine A couplée par bandelette a été estimée à 0,8 µg.

EXEMPLE 4 : préparation d'une bandelette de diagnostic portant un haptène

a) Caséine-spermine

Tout d'abord la caséine a été couplée dans les conditions identiques à celles décrites pour la protéine A et ensuite la spermine a été couplée à la caséine dans les conditions suivantes :

2 bandelettes ont été incubées dans 500 µl de $NaH_2PO_4$ 0,01 M pH 5 contenant un carbodiimide hydrosoluble (1 éthyl 3,3 diméthyl-aminopropyl carbodiimide) à 0,1 M.

Après 10 minutes de contact la spermine a été ajoutée à une concentration finale de 0,1 M, contact suivi d'un changement du pH à pH 8,5 à l'aide de borate de sodium 0,1 M (Davis et Preston, 1981, 116, 402-407).

L'utilisation de spermine $^{14}C$ a permis de déterminer que la quantité de spermine fixée par bandelette est de l'ordre de 10 nmoles.

EXEMPLE 5 : préparation d'une bandelette de diagnostic portant de l'ADN

i) Préparation des fragments d'ADN.

L'ADN de thymus de veau (Boehringer) a été digéré avec de l'endonucléase $ECOR_1$ (Boehringer) à raison de 2 unités d'enzyme/µg d'ADN dans un tampon contenant NaCl 0,05 M, $MgCl_2$ 0,01 M, Tris-HCl 0,01 M pH 7,5.

Après digestion pendant 2 h à 37°C, la réaction a été arrêtée par abaissement de la température à 0°C et en ajoutant de l'EDTA froid à une concentration finale de 0,02 M. L'ADN digéré a été dénaturé par chauffage à 65°C pendant 10 minutes suivi d'un refroidissement rapide à 0°C. Les fragments ont été précipités tout d'abord avec de la spermine (Sigma) à une concentration finale de 0,02 M dans un tampon contenant NaCl 0,01 M, Tris HCl 0,01 M pH 7,5.

Après deux lavages dans l'eau distillée le précipité a été dissous dans du NaCl 1 M.

La concentration de NaCl a été ramenée à 0,01 M et l'ADN précipité par addition d'éthanol 95% pendant une nuit à -20°C.

Après centrifugation à 17000 g pendant 45 minutes le culot a été séché et redissous finalement dans un tampon contenant : EDTA 0,01 M, $NaH_2PO_4/KH_2PO_4$ 0,01 M pH 8,0.

La détermination de la densité optique à 260 nm a permis de calculer le rendement de la préparation des fragments d'ADN. Il était de 50% à 75% de l'ADN de départ (4 préparations).

La taille des fragments a été contrôlée par électrophorèse en gel d'agarose.

ii) Couplage des fragments d'ADN sur bandelette-AH.

Le couplage a été effectué sur les bandelettes diazotées selon la technique décrite précédemment.

200 µg d'ADN (fragments) on été mis en contact avec 10 bandelettes diazotées.

La quantité d'ADN couplé par bandelette a été estimée à 0,8 µg.

<u>EXEMPLE 6 : préparation d'un réactif de diagnostic par couplage d'Ag virus cytomégalique CMV sur des plaques (en polystyrène) de microtitration à 96 puits :</u>

(A) Conversion du polystyrène en polyamino-styrène

(B) Introduction de bras latéraux terminant en acide-hydrazide

(C) Couplage proprement dit de l'antigène viral

(A) La conversion du polystyrène en polyamino-styrène a été accomplie selon la technique décrite par CHIN & LINKS (Anal. Biochem, 1977, 83, 709-719). La technique suivie est exactement celle décrite par ces auteurs. Sa mise en oeuvre a permis de substituer par puits 1 nmol équivalent de $NH_2$ (éthanolamine comme étalon).

(B) L'introduction de bras latéraux terminant en acide-hydrazide a été accomplie selon la technique décrite dans le Brevet INSERM 2 450 877. La détermination du nombre de groupements d'acide-hydrazide présent par puits à l'aide de l'acide picryl-sulfonique a révélé la présence de 1 nmol d'acide-hydrazide par puits.

(C) Couplage proprement dit de l'antigène CMV.
La préparation de l'antigène CMV (AgCMV) et son couplage ont été effectués selon les techniques décrites dans l'Exemple 1 ci-dessus.
Pour l'AgCMV et l'$AgMRC_5$ la quantité de protéine fixée par puits était de l'ordre de 1 µg.

B. PREPARATION DE BANDELETTES-ANTICORPS

<u>EXEMPLE 7 : préparation d'une bandelette de réactif portant un anticorps anti-Salmonella</u>

Couplage proprement dit :
0,5 mg d'immunoglobulines ont été préalablement oxydés conformément aux modalités décrites dans le Brevet Français INSERM 2 331 567.
Les immunoglobulines ainsi oxydées ont été mises en contact avec les bandelettes AH à raison de 20 µg/bandelette.
La quantité de protéine couplée par bandelette (4 mm x 8 mm) était de l'ordre de 1 µg.

<u>EXEMPLE 8 : saturation des bandelettes avant leur utilisation dans la méthode ELISA</u>

Afin d'éliminer l'adsorption non spécifique de protéines sériques lors de la réaction ELISA, les bandelettes "Nylon" et "Cellulose" ont été saturées avec des protéines telles que la sérumalbumine bovine, les IgG de veau, la gélatine. Le choix

de la protéine et de sa concentration dépend de l'antigène couplé.

Préparation de réactifs particuliers pour la mise en oeuvre de la méthode ELISA avec des bandelettes-anticorps :

EXEMPLE 9 : préparation d'anticorps anti-Salmonella biotinylés

0,3 mg d'immunoglobulines ont été préalablement oxydés conformément aux modalités décrites dans le Brevet Français INSERM 2 331 567. 0,2 µmole de biotine-hydrazide (Sigma) ont été ajoutés aux anticorps oxydés en solution dans un tampon contenant : NaCl 0,14 M, $Na_2H\ PO_4/KH_2PO_4$ 0,01 M pH 6.

La solution d'anticorps oxydés et de biotine-hydrazide a été refroidie à 0°C, le pH ajusté à pH 2 avec HCl 0,1 M. Après 10 minutes de contact, le pH a été ramené à pH 6 avec une solution de $Na_2HPO_4$ 0,4 M.

Après un contact de 2 h à 4°C, l'excès de biotine-hydrazide a été éliminé par dialyse contre un tampon phosphate ($Na_2HPO_4/KH_2PO_4$)0,01 M pH 7,5.

C. EXEMPLES DE REACTIONS ANTIGENE-ANTICORPS A L'AIDE DE BANDELETTES ANTIGENES ET DE PLAQUES ANTIGENES

EXEMPLE 10 : réaction entre une bandelette-antigène et des anticorps marqués directement avec une enzyme

Les bandelettes utilisées sont les suivantes :

1. Expérimental : il s'agit des bandelettes"Nylon"-AH-CHO-IgG de l'Exemple 2.
2. Contrôle : il s'agit des bandelettes "Nylon"-AH obtenues au 1. de l'Exemple 2.

A. Les bandelettes "Expérimental" et "contrôle" sont immergées pendant 20 minutes dans 200 µl d'une solution de sérum de lapin contenant des anticorps anti-IgG humaines marqués à la péroxydase (POD), diluée au 1/250è dans un tampon qui présente la composition suivante :

| | | |
|---|---|---|
| 0,14 | M | NaCl |
| 0,1 | M | glycine |
| 0,03 | M | Borate de Na |

0,1% (v/v) "Pluronic" L62 (détergent non-ionique, ajusté à pH 8,1, par 1,0 M HCl).

A la fin du temps de contact indiqué plus haut, les bandelettes sont lavées par trois jets successifs du tampon ci-dessus, à l'aide d'une pissette, en égouttant entre chaque lavage.

B. Les lavages effectués, les bandelettes sont immergées dans 200 µl d'une solution contenant, pour 6 ml :

0,1 M acétate de sodium-acide acétique pH 5,

6 mg α-chloronaphtol en solution dans 1 ml méthanol,

200 µl $H_2O_2$ à 0,3% dans l'eau.

Après 15 minutes de contact, une coloration pourpre se développe sur les bandelettes "Nylon"-AH-CHO-IgG dites "Experimental", tandis que les bandelettes "Contrôle" "Nylon"-AH restent blanches. Les surnageants ne sont colorés dans aucun des deux cas.

L'utilisation décrite précédemment de bandelettes antigènes pour détecter des anticorps marqués soit par une enzyme soit par la biotine n'est pas limitative.

En effet, la réaction entre les anticorps marqués et l'antigène couplé sur la bandelette peut être inhibée soit en incubant préalablement la bandelette antigène avec un sérum contenant des anticorps non marqués, soit en préincubant les anticorps marqués avec le prélèvement contenant l'antigène recherché.

EXEMPLE 11 : dosage d'anticorps selon la technique d'inhibition

Les anticorps anti CMV

Pour le dosage des anticorps anti CMV neutralisants présents dans un sérum, une bandelette "Nylon"-CMV est mise en contact avec le sérum à tester. Dans un deuxième temps et après lavage avec un tampon de lavage dit "Tampon II" présentant de préférence la composition suivante :

TAMPON II

Sérum albumine bovine .................. 1,0 %
NaCl ..................................... 0,3 M
Glycine .................................. 0,1 M
Borate de sodium ......................... 0,05 M
"Synpéronic" PE/L62 (détergent) ....... 0,10 % v/v

la bandelette est mise en contact avec les anticorps anti CMV marqués.

Pour les sérums ayant un titre en anticorps anti-CMV élevé, la coloration est plus faible que celle obtenue avec les sérums ayant un titre en anticorps anti-CMV faible.

<u>EXEMPLE 12 : Détermination du titre en anticorps anti CMV neutralisants sur les plaques portant l'AgCMV et l'AgMRC5 couplés par leurs résidus glucidiques.</u>

a) Saturation des plaques avant leur utilisation en ELISA

Afin d'éliminer l'adsorption non spécifique des protéines sériques lors de la réaction ELISA, les plaques de microtitration ont été saturées par des IgG de veau à 0,5 % en solution dans le NaCl 0,14 M à raison de 200 µl par puits.

b) Mise en évidence d'IgG anti-CMV dans le sérum humain

Le sérum est dilué dans un tampon I de composition suivante :

IgG veau .. 0,5 % Borate de sodium ... 0,05M
NaCl ...... 0,14M Synpéronic PE/L62 .. 0,10%(v/v)
Glycine ... 0,1 M

Cette solution est ajustée à pH 8,1 par 1,0 M HCl.

100 µl de chaque dilution sont répartis dans les puits de la plaque contenant l'antigène CMV et l'antigène $MRC_5$. Après 2 heures d'incubation à 37°C, les plaques sont lavées dans le tampon décrit précédemment mais sans lgG de veau.

Dans chaque puits sont alors introduits 100 µl d'une

solution de sérum de chèvre contenant des anticorps anti-IgG humaines marqués à la phosphatase alcaline, dilué au 1/1000 dans le tampon II suivant :

Sérum albumine bovine .... 1,0 % (p/v)
NaCl .................... 0,14M
Glycine .................. 0,1 M
Borate ................... 0,05M
Synpéronic PE/L62 ........ 0,10% (v/v)

Après une heure de contact à 37°C et lavage dans le tampon précité mais sans sérum-albumine bovine, l'activité enzymatique est révélée à 37°C à l'aide du substrat p-nitrophénylphosphate à la concentration de 0,2% (p/v) en solution dans un tampon :

Aminométhylpropanol ... 0,625 M pH 10,25
$MgCl_2$ ................ 2,0 mM

La densité optique est mesurée à 405 nm toutes les 5 minutes pendant 30 minutes ou bien une seule lecture de densité optique est effectuée après une incubation de 30 minutes.

c) Comparaison des titres en IgG anti-CMV mesurés par trois techniques différentes sur des fractions IgG purifiées à partir de sérums humains

1) Test ELISA classique dans lequel les antigènes CMV et $MRC_5$ sont fixés par adsorption sur plaque de PVC,
2) Test de neutralisation de pouvoir infectieux du virus (CMV) sur culture de cellules $MRC_5$,
3) Test ELISA covalent décrit ci-dessus pour les sérums humains.

Après détermination des titres par les trois techniques de dosage, ces titres sont ramenés à une concentration de protéines de 1 mg/ml dans la préparation non diluée de $\gamma$-globulines comme le montre le tableau 3 qui va suivre.

TABLEAU 3

| γ-globulines du CTS n° | Titres 1 ELISA adsorption | Titres 2 Neutralisation | Titres 3 ELISA covalent (résidus glucidiques) |
|---|---|---|---|
| Y0504 | 67 | 128 | 50 |
| Y0714 | 144 | 256 | 50 |
| Y0880 | 519 | 256 | 100 |
| Y0969 | 1087 | 512 | 400 |
| Y1042 | 54 | 64 | <50 |
| Y0741 | 67 | 128 | 50 |
| Y0287 | 432 | 256 | 200 |
| Y0417 | 2780 | 465 | 364 |
| Y0454 | 1005 | 621 | ≥727 |
| S1416 | 2923 | 512 | 400 |
| | $r_1 = 0,720$ | $r_2 = 0,934$ | |

Il ressort de ce Tableau 3 qu'il existe une bonne corrélation entre les titres obtenus en ELISA covalent et ceux obtenus en neutralisation ($r_2 = 0,934$). Par contre, entre les titres en ELISA par adsorption et ceux obtenus en neutralisation la corrélation n'est pas aussi bonne ($r_1 = 0,720$).

EXEMPLE 13 : dosage d'antigène selon la technique d'inhibition

La spermine

Pour le dosage du taux de spermine présente dans les liquides biologiques (sérum, urine et LCR) les bandelettes "Nylon"-caséine-spermine ou Nitrocellulose-caséine-spermine sont utilisées.

La réaction se déroule de la manière suivante : un aliquot du liquide biologique est incubé avec les anticorps anti-spermine marqués. Après 30 minutes d'incubation, les anticorps marqués ayant réagi avec la spermine ne sont plus disponibles pour la réaction avec la caséine-spermine présente sur la bandelette. Par contre, les anticorps marqués n'ayant pas rencontré la spermine peuvent réagir.

Ainsi, la diminution de l'intensité de la coloration due à l'enzyme fixée est une mesure de la quantité de spermine présente dans le prélèvement.

On peut utiliser les réactions de compétition (similaires à celles décrites ci-dessus) entre les antigènes sériques et les mêmes antigènes immobilisés sur des bandelettes, pour le dosage de toutes les protéines sériques pour lesquelles les anticorps existent.

EXEMPLE 14 : mise en évidence d'anticorps présents dans un sérum humain à l'aide de bandelettes Ag

Le sérum est dilué dans un tampon I de composition suivante :

| | |
|---|---|
| IgG veau ... 0,5 % | Borate de sodium ... 0,05 M |
| NaCl ....... 0,3 M | "Synpéronic" |
| Glycine .... 0,1 M | PE/L62 ........... 0,10 % (v/v) |

Cette solution est ajustée à pH 8,1 par 1,0 M HCl. 200 µl de chaque dilution sont répartis dans les puits d'une microplaque. Dans chaque puits est insérée une bandelette "expérimentale" contenant l'antigène à tester ou une bandelette "contrôle". Après 2 h de contact à 37°C les bandelettes sont enlevées et lavées par trois jets successifs du tampon

précité, mais sans IgG de veau, à l'aide d'une pissette, en égouttant entre chaque lavage.

Les bandelettes "expérimentales" et "contrôles" sont immergées pendant 20 minutes dans 200 µl d'une solution de sérum de chèvre contenant des anticorps anti-IgG humaines marqués à la peroxydase (POD), diluée au 1/250è dans le tampon II décrit ci-dessus.

A la fin du temps de contact indiqué plus haut, les bandelettes sont lavées par trois jets successifs du tampon II, mais sans sérum-albumine bovine, en égouttant entre chaque lavage.

Les lavages effectués, les bandelettes sont immergées dans 200 µl d'une solution contenant pour 6 ml : $Na_2HPO_4/KH_2PO_4$ 0,01 M pH 6, 200 µl $H_2O_2$ à 0,3 % dans l'eau, $\alpha$ -chloronaphtol 6 mg dans 1 ml méthanol.

Après 15 minutes de contact, une coloration pourpre se développe sur les bandelettes "Expérimentales" tandis que les bandelettes "contrôles" restent blanches.

Les surnageants ne sont colorés dans aucun des deux cas.

Par comparaison avec une échelle de coloration établie sur des bandelettes ayant des quantités variables d'IgG fixées, on calcule la quantité d'anticorps ayant réagi avec la bandelette.

La technique décrite ci-dessus a été employée pour déterminer le taux d'anticorps 1) anti-CMV, 2) anti-polyamines, 3) anti-ADN et le taux d'IgG humaines réagissant avec la protéine A (Voir Tableau 4 ci-après).

Dans le cas des anticorps anti-spermine, un sérum de lapin anti-IgG de chèvre marqué à la phosphatase alcaline a été utilisé. L'activité enzymatique est révélée à l'aide du substrat p-nitro phényl phosphate (Technicon).

## - TABLEAU 4 -

| | | BANDELETTE | Groupement utilisé pour le couplage | Interaction avec: ANTICORPS | Interaction avec: ANTIGENES | NATURE DES CONJUGUES | REACTION | RESULTATS |
|---|---|---|---|---|---|---|---|---|
| ANTIGENES macromoleculaires | Viral | -$MRC_5$ infectées CMV | CHO (sucre) | sérums humains | --- | chèvre anti IgG humaine -POD | Ind. | Figure 3 (A,B,E,F) |
| | Controle | -$MRC_5$ non infectées | CHO " | " | --- | " | | Figure 3 (C,D,G,H) |
| | Protéine sérique | IgG humaines | CHO " | --- | --- | Chèvre anti IgG humaine -POD | Dir. | Figure 4 (A,C) |
| | Contrôle | -AH | ----- | --- | --- | " | | Figure 4 (B,D) |
| | Bactérien | Protéine A de S. aureus | $\varepsilon$-$NH_2$ | IgG humaines | --- | chèvre anti IgG humaine -POD | Ind. | Figure 5 (A) |
| | Contrôle | AH-éthanolamine | | " | | " | | Figure 5 (B) |
| HAPTENES | Spermine | -Caséine-Spm | $\varepsilon$-$NH_2$ caséine-COOH-Spm | chèvre anti Spm | --- | lapin anti IgG chèvre -PA | Ind. | Tableau 4 |
| | Contrôle | -Caséine | $\varepsilon$-$NH_2$ caséine | " | --- | " | | Tableau 4 |
| | Acide nucléique | -ADN | $NH_2$ $C^6$ adénine | sérum humain | --- | chèvre anti IgG humaine -POD | Ind. | Figure 6 (A) |
| | Contrôle | AH-éthanolamine | | " | --- | " | | Figure 6 (B) |
| ANTICORPS | Ac | anti Salmonella 04-5 | CHO (sucre) | --- | suspension bactérienne | 1er: anti Salmonella biotine; 2ème: Streptavidine -POD | Ind. | Figure 7 (A) |
| | Contrôle | | " | --- | " | 1er: ----; 2ème: " | | Figure 7 (B) |

Spm : Spermine ; AH : Acide-Hydrazide ; POD : Peroxydase ; PA : Phosphatase alcaline ; Dir : Directe ; Ind : Indirecte.

Dans le tableau 4 ci-dessus, la figure 3 à laquelle il est fait référence dans la colonne "résultats" et qui concerne l'antigène viral CMV, comporte une première ligne relative à AgCMV et une deuxième ligne relative au contrôle AgMRC5 :

- en relation avec AgCMV, A, B, E et F désignent des bandelettes "Nylon"-AH sur lesquelles ont été fixées les protéines provenant de l'homogénéisat de cellules MRC5 infectées par le cytomégalovirus ;
- C, D, G et H désignent des bandelettes de "Nylon"AH sur lesquelles ont été fixées les protéines provenant de l'homogénéisat de cellules MRC5 non infectées (contrôle).

Pour les bandelettes A, B, C et D, on a utilisé du sérum humain contenant des IgG anti-CMV et pour les bandelettes E, F, G et H on a utilisé du sérum humain ne contenant pas d'IgG anti-CMV.

La figure 4 représente les résultats obtenus avec une protéine sérique (IgG) :

- la lettre A se réfère à une bandelette "Nylon"-AH sur laquelle ont été fixées des IgG humaines ;
- la lettre B se réfère à une bandelette "Nylon"-AH de contrôle ;
- la lettre C se réfère à une bandelette de Nitrocellulose-AH sur laquelle ont été fixées des IgG humaines ;
- la lettre D se réfère à une bandelette de Nitrocellulose-AH de contrôle.

La figure 5 représente les résultats obtenus avec un antigène bactérien (protéine A) :

- dans cette figure, A désigne une bandelette de Nitrocellulose-AH sur laquelle a été fixée la protéine A ,
- et B désigne une bandelette de Nitrocellulose-AH sur laquelle a été fixée de l'éthanolamine.

La figure 6 à laquelle il est fait référence dans la colonne "résultats" concerne les résultats obtenus avec un haptène (ADN) :

- A désigne une bandelette de Nitrocellulose-AH sur laquelle a été fixé de l'ADN ;
- et B désigne une bandelette de Nitrocellulose-AH sur laquelle a été fixée de l'éthanolamine.

La figure 7 concerne les résultats obtenus dans le cas d'une bactérie entière (Salmonella) :
- A et B désignent des bandelettes "Nylon"-AH, traitées par la streptavidine-peroxydase, sur lesquelles les anticorps anti-Salmonella ont été fixés et mis en contact avec une suspension de Salmonella.

A désigne une bandelette traitée avec des anticorps anti-Salmonella biotinylés tandis que la bandelette B n'a pas été traitée par ces anticorps.

Le tableau 5 auquel il est fait référence dans le tableau 4 ci-dessus (résultats) concerne un haptène particulier, à savoir la spermine.

TABLEAU 5

| DO (405 nm) | Nylon-AH | Nitrocellulose-AH |
|---|---|---|
| caséine-spermine | 1,24 | 1,10 |
| caséine (contrôle) | 0,75 | 0,12 |

D. MISE EN EVIDENCE D'UN ANTIGENE PRESENT DANS UN PRELEVEMENT A L'AIDE DE BANDELETTES-ANTICORPS

EXEMPLE 15 : mise en évidence d'un antigène bactérien Salmonella typhimurium

Tampon de suspension des germes

200 µl d'une suspension de germes correspondant à $6.10^7$ germes/ml et contenant 18 µg protéine/ml sont répartis dans les puits d'une microplaque. Dans chaque puits est insérée soit une bandelette "Expérimentale" soit une bandelette "contrôle". Après 1 h de contact à 37°C les bandelettes sont lavées par trois jets successifs du même tampon mais dépourvu d'IgG de veau, à l'aide d'une pissette, en égouttant entre chaque lavage.

Aux bandelettes expérimentales sont ajoutés des anticorps biotinylés. Les bandelettes "contrôles" ne reçoivent pas d'anticorps biotinylés.

Après 1 h de contact à 37°C et trois lavages selon la technique décrite ci-dessus, les bandelettes expérimentales et contrôles sont mises en contact avec de la streptavidine-peroxydase (Sigma) en solution à 0,5 µg protéine par ml de tampon II.

Après 3 lavages avec ce même tampon mais sans sérum-albumine bovine, l'activité POD est révélée comme décrit précédemment.

Les réactifs de diagnostic conformes à la présente invention permettent une détection qualitative et quantitative très sensible d'anticorps ou d'antigènes et notamment d'anticorps neutralisants, dans un fluide biologique, en même temps qu'une lecture facile.

Ainsi que cela ressort de ce qui précède, l'invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée, de la présente invention.

REVENDICATIONS

1. Réactif de diagnostic, caractérisé en ce qu'il comprend essentiellement un support solide pris dans le groupe qui englobe les microplaques en polystyrène ou en PVC et les bandelettes, lequel support porte des chaînes latérales constituées par un dérivé d'hydrazine et/ou un acide aminé, auxquelles sont fixées par liaison chimique, des molécules d'antigène ou d'anticorps.

2. Réactif de diagnostic selon la revendication 1, caractérisé en ce que le support solide est constitué par une bandelette qui se compose d'une couche de matière textile appropriée fixée à une couche de matière thermoplastique telle que notamment le PVC ou le polystyrène.

3. Réactif de diagnostic selon la revendication 2, caractérisé en ce que la couche en matière textile qu'il comprend est choisie parmi les fibres de polyamide, les matériaux à base de cellulose tels, notamment, que la cellulose, la nitrocellulose, l'acétate de cellulose, les polyesters et analogues.

4. Réactif de diagnostic selon l'une quelconque des revendications 1 à 3, caractérisé en ce que les chaînes latérales fixées au support solide, sont constituées par un composé pris dans le groupe qui comprend des dérivés d'hydrazine tels que les acides-hydrazides et la phénylhydrazine et les acides aminés, notamment les acides aminés contenant un groupe -SH.

5. Réactif de diagnostic selon l'une quelconque des revendications 1 à 4, caractérisé en ce que les molécules fixées par liaison chimique aux chaînes latérales portées par le support solide, sont prises dans le groupe qui comprend les antigènes viraux, les antigènes bactériens, les haptènes, les anticorps, les protéines plasmatiques, les acides ribonucléiques et désoxyribonucléiques, les immunoglobulines utilisables selon le cas comme antigènes ou anticorps.

6. Réactif de diagnostic selon l'une quelconque des

revendications 1 à 5, caractérisé en ce que les antigènes ou anticorps fixés par liaison chimique aux chaînes latérales portées par le support solide sont constitués par des protéines ou des glycoprotéines non oxydées et lesdites chaînes latérales sont constituées par un acide-hydrazide diazoté ou par un composé organique comportant un groupe carboxyle terminal.

7. Réactif de diagnostic selon l'une quelconque des revendications 1 à 5, caractérisé en ce que les antigènes ou anticorps fixés par liaison chimique aux chaînes latérales portées par le support solide sont constitués par des glycoprotéines oxydées et lesdites chaînes latérales sont constituées par un composé pris dans le groupe qui comprend des dérivés d'hydrazine tels que les acides-hydrazides et la phénylhydrazine et les acides aminés, notamment les acides aminés contenant un groupe -SH, tels que la cystéine, en particulier.

8. Réactif de diagnostic selon la revendication 7, caractérisé en ce qu'il comprend un antigène du virus cytomégalique (CMV) préalablement oxydé et fixé par couplage chimique par ses résidus glucidiques, aux chaînes latérales constituées par un dérivé d'hydrazide, portées par le support solide tel que microplaque en PVC ou en polystyrène ou bandelette en fibres de matière textile elle-même fixée sur un support en matière thermoplastique telle que PVC ou polystyrène notamment.

9. Réactif de diagnostic selon l'une quelconque des revendications 1 à 8, caractérisé en ce que, dans le cas où le support solide est une bandelette, la couche de matière textile est avantageusement fixée sur la couche de matière thermoplastique inerte, par soudage, et de préférence par soudage haute fréquence.

10. Réactif de diagnostic selon la revendication 9, caractérisé en ce que la couche de matière textile est en matériau à base de cellulose, oxydé.

11. Réactif de diagnostic selon l'une quelconque des revendications 9 et 10, caractérisé en ce que la couche de matière textile est fixée à une partie seulement de la couche de matière thermoplastique.

12. Réactif de diagnostic selon la revendication 11, caractérisé en ce que la surface de la partie de la couche de matière plastique à laquelle est fixée la couche de matière textile à laquelle sont fixés des antigènes ou des anticorps par l'intermédiaire de chaînes latérales, est comprise entre 1/5 et 1/50 environ de la surface totale de la couche de matière thermoplastique.

13. Réactif de diagnostic selon l'une quelconque des revendications 1 à 12, caractérisé en ce qu'il présente sensiblement la forme d'une bandelette.

14. Réactif de diagnostic selon la revendication 13, caractérisé en ce qu'une pluralité de bandelettes sont associées entre elles par l'intermédiaire d'une bande de support commune.

15. Réactif de diagnostic selon la revendication 14, caractérisé en ce que les bandelettes présentent une ligne d'affaiblissement à la jonction de chacune d'entre elles à la bande de support commune.

16. Réactif de diagnostic selon l'une quelconque des revendications 1 à 12, caractérisé en ce que le support solide ou la couche de support de la bandelette en matière thermoplastique est en polystyrène et en ce que celui-ci est traité au préalable pour le convertir en polyaminostyrène.

17. Procédé de production du réactif de diagnostic selon l'une quelconque des revendications 1 à 16, caractérisé en ce que le support solide qu'il comprend comporte des groupes amine primaire libres, en ce que ledit support est substitué par des chaînes latérales par couplage de celles-ci avec lesdits groupes $NH_2$ libres et en ce que des molécules d'un antigène ou d'un anticorps approprié sont fixées par liaisons de covalence aux extrémités libres des chaînes latérales préalablement fixées par couplage sur le support solide.

18. Procédé de production du réactif de diagnostic selon la revendication 17, caractérisé en ce que dans le cas où le support solide est constitué par une bandelette dans laquelle une couche de matière textile est associée à une couche de matière thermoplastique, ledit procédé comprend :

- une première étape au cours de laquelle une couche faite de fibres d'une matière textile appropriée est substituée par des chaînes latérales couplées aux groupements $NH_2$ libres des fibres de matière textile ;

- une deuxième étape au cours de laquelle des molécules d'un antigène ou d'un anticorps convenable sont fixées par liaisons de covalence aux extrémités libres des chaînes latérales fixées à la couche de fibres de matière textile au cours de la première étape.

19. Procédé selon l'une quelconque des revendications 17 ou 18, caractérisé en ce que le support comportant des groupes $NH_2$ libres, est substitué par des chaînes latérales d'un dérivé d'hydrazine, par succinylation desdits groupes $NH_2$ par de l'anhydride succinique, suivie de la transformation de la fonction acide terminale en hydrazide, par réaction avec l'hydrazine, en présence d'un carbodiimide approprié.

20. Procédé selon l'une quelconque des revendications 17 ou 18, caractérisé en ce que le support comportant des groupes $NH_2$ libres, est substitué par des chaînes latérales d'un acide aminé comportant un groupe -SH, notamment par la cystéine, en présence d'un carbodiimide approprié.

21. Procédé selon l'une quelconque des revendications 17 à 20, caractérisé en ce que l'antigène ou l'anticorps comporte un résidu glucidique comprenant au moins un groupe $-CH_2OH$, qui est oxydé en un groupe -CHO préalablement à la fixation de l'antigène ou de l'anticorps à une chaîne latérale du support solide.

22. Procédé selon la revendication 21, caractérisé en ce que l'antigène ou l'anticorps est fixé au support par réaction chimique entre son groupe -CHO terminal et le groupe amine primaire libre terminal porté par la chaîne latérale du support solide.

23. Procédé selon l'une quelconque des revendications 17 à 22, caractérisé en ce qu'il comprend l'association d'une bandelette de matière textile à une couche de matière thermoplastique.

24. Procédé selon la revendication 23, caractérisé en ce que la bandelette de matière textile est en fibres de polyamide.

25. Procédé selon la revendication 23, caractérisé en ce que la bandelette de matière textile est en fibres de polyester.

26. Procédé selon l'une quelconque des revendications 17 à 22, caractérisé en ce que la bandelette de matière textile est en fibres de matériau à base de cellulose pris dans le groupe de la cellulose, de la nitrocellulose et de l'acétate de cellulose.

27. Procédé selon la revendication 26, caractérisé en ce que la couche en fibres de matériau à base de cellulose est soumise à un processus d'oxydation préalablement à la fixation des chaînes latérales de dérivé d'hydrazine.

28. Procédé selon l'une quelconque des revendications 26 et 27, caractérisé en ce qu'après la fixation de chaînes latérales de dérivé d'hydrazine sur la couche oxydée, les groupes carbonyle libres qui n'ont pas réagi au cours de l'étape de fixation des chaînes latérales, avec le dérivé d'hydrazine, sont réduits en alcools par un agent réducteur approprié tel que, notamment, le borohydrure de sodium.

29. Procédé selon l'une quelconque des revendications 17 à 20 et 23 à 28, caractérisé en ce que l'antigène ou l'anticorps est fixé au support solide par réaction chimique

entre son groupe-$NH_2$ et le groupe acide-hydrazide de la chaîne latérale fixée audit support.

30. Procédé selon la revendication 29, caractérisé en ce que l'antigène ou l'anticorps fixé par couplage chimique est pris dans le groupe des protéines, des holoprotéines, des glycoprotéines.

31. Procédé selon l'une quelconque des revendications 29 et 30, caractérisé en ce que les chaînes latérales du support solide, constituées par un acide-hydrazide, sont diazotées en un acide-azide auquel sont couplés par réaction chimique des antigènes ou des anticorps constitués par des protéines, des holoprotéines ou des glycoprotéines, ainsi que par des acides ribo- et desoxyribonucléiques, non oxydés.

32. Procédé selon l'une quelconque des revendications 23 à 28, caractérisé en ce que la couche en fibres de matière textile est fixée à la couche de matière thermoplastique préalablement à la fixation des chaînes latérales et de l'antigène ou l'anticorps anti-espèce, ou postérieurement à ces étapes de traitement.

33. Procédé de détection d'anticorps ou d'antigènes dans un fluide biologique tel que sérum, caractérisé en ce qu'il comprend :

- une première étape au cours de laquelle un réactif de diagnostic selon l'une quelconque des revendications 1 à 16, est immergé pendant un temps approprié, dans une solution de sérum ;
- une deuxième étape au cours de laquelle le réactif de diagnostic retiré de la solution de sérum, puis lavé, est introduit dans une solution contenant des anticorps anti-espèce à détecter, marqués par une enzyme, puis est lavé à plusieurs reprises à l'aide d'un tampon de composition convenable ;
- une troisième étape au cours de laquelle le réactif de diagnostic est introduit dans une solution contenant un substrat avantageusement associé à un accepteur d'électrons, qui précipite sur ledit réactif, en présence d'anticorps spécifiques, en donnant à celle-ci une coloration pourpre plus ou moins intense selon la quantité de produit formé, laquelle coloration, par comparaison à une échelle de colorations standard, mesure la quantité d'anticorps présente dans le fluide biologique testé.

34. Procédé de détection selon la revendication 33, caractérisé en ce que les molécules fixées par couplage chimique aux chaînes latérales constituées par un dérivé d'hydrazine, du réactif de diagnostic, sont des molécules d'immunoglobulines (IgG, IgM, IgE) préalablement oxydées, couplées par réaction chimique de la fonction $-NH_2$ libre d'une chaîne latérale de dérivé d'hydrazine, avec la fonction -CHO libre de la molécule d'immunoglobuline-CHO correspondante, avec formation d'hydrazone.

35. Procédé de détection selon l'une quelconque des revendications 33 ou 34, caractérisé en ce que les anticorps dirigés contre l'antigène à détecter sont marqués directement par une enzyme et sont introduits dans le fluide biologique à tester, dans une dilution d'un tampon approprié contenant un détergent non-ionique, du borate de sodium, un acide aminé approprié et du chlorure de sodium, ajusté à pH 8,1 environ.

36. Procédé de détection selon l'une quelconque des revendications 33 à 35, caractérisé en ce que les molécules fixées par couplage chimique aux chaînes latérales constituées par un dérivé d'hydrazide, du support solide du réactif de diagnostic, sont des antigènes viraux, notamment des antigènes du virus cytomégalique (CMV), préalablement oxydés.

37. Procédé de détection selon l'une quelconque des revendications 33 et 35, caractérisé en ce que les molécules fixées par couplage chimique aux chaînes latérales constituées par un dérivé d'hydrazide, du support du réactif de diagnostic, sont des antigènes bactériens tels que, notamment, la protéine A provenant de Staphylococcus aureus, couplée chimiquement aux chaînes latérales diazotées de la couche de support.

38. Procédé de détection selon l'une quelconque des revendications 33 et 35, caractérisé en ce que les molécules fixées par couplage chimique aux chaînes latérales constituées par un dérivé d'hydrazide, du support du réactif de diagnostic, sont un haptène, notamment un produit de couplage caséine-spermine couplé chimiquement aux chaînes latérales diazotées dudit support.

39. Procédé de détection selon l'une quelconque des revendications 33 et 35, caractérisé en ce que les molécules fixées par couplage chimique aux chaînes latérales constituées par un dérivé d'hydrazide, du support du réactif de diagnostic, sont un acide désoxyribonucléique, couplé chimiquement aux chaînes latérales diazotées de la couche de support.

40. Procédé de détection selon l'une quelconque des revendications 33 et 35, caractérisé en ce que les molécules fixées par couplage chimique aux chaînes latérales constituées par un dérivé d'hydrazide, du support du réactif de diagnostic, sont des anticorps pris, entre autres, dans le groupe qui comprend les anticorps anti-Salmonella préalablement oxydés et biotinylés.

41. Procédé de détection selon l'une quelconque des revendications 33 à 40, caractérisé en ce que le tampon utilisé pour le lavage des réactifs de diagnostic à leur sortie du fluide biologique, est le même que celui utilisé pour la dilution des anticorps anti-espèce, marqués par une enzyme.

42. Procédé de détection selon l'une quelconque des revendications 33 à 41, caractérisé en ce que le substrat est constitué soit par une solution de peroxyde d'hydrogène associé à de l' $\alpha$-chloronaphtol ou à l'o-dianisidine, ou encore à l'o-phénylènediamine, soit par une solution de p-nitrophénylphosphate, notamment.

43. Procédé de détection selon l'une quelconque des revendications 33 à 42, caractérisé en ce que les réactifs de diagnostic selon l'une quelconque des revendications 1 à 16

sont saturés, préalablement à leur mise en oeuvre dans ledit procédé de détection, par des protéines prises, notamment, dans le groupe qui comprend la sérum-albumine bovine, les IgG de veau, la gélatine, dans le but d'éliminer l'adsorption non spécifique de protéines sériques pendant le procédé de détection par la réaction ELISA.

44. Coffret, ou kit, de diagnostic prêt à l'emploi pour la réalisation du procédé de détection selon l'une quelconque des revendications 33 à 43, caractérisé en ce qu'il comprend, en combinaison :

- un réactif de diagnostic selon l'une quelconque des revendications 1 à 16, portant des antigènes ou des anticorps appropriés ;
- une quantité appropriée d'anticorps marqués par une enzyme, dirigés contre l'antigène du réactif de diagnostic ;
- une quantité appropriée de tampon I ;
- une quantité appropriée de tampon II ;
- une pluralité de plaques de microtitration ;
- une quantité appropriée de substrat de l'enzyme ;
- une quantité appropriée d'accepteur d'électrons.

45. Coffret de diagnostic selon la revendication 44, caractérisé en ce que le réactif de diagnostic se présente sous forme de bandelettes unitaires ou de bandelettes multiples.

46. Coffret de diagnostic selon la revendication 44, caractérisé en ce que le réactif de diagnostic selon l'une quelconque des revendications 1 à 16, portant des antigènes ou anticorps appropriés, est fixé par couplage chimique aux chaînes latérales constituées par un dérivé d'hydrazide portées par des plaques de microtitration et plus spécifiquement fixées dans chacun des puits desdites plaques.

FIG. 1

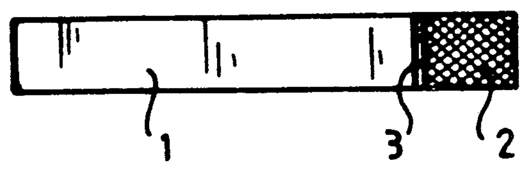


FIG. 2

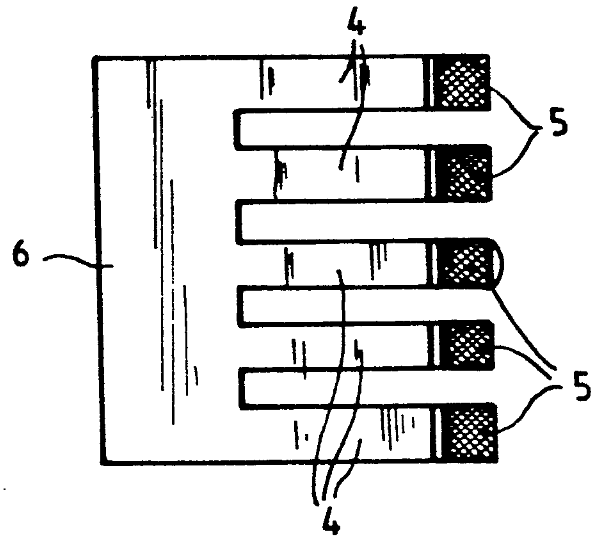

FIG.3

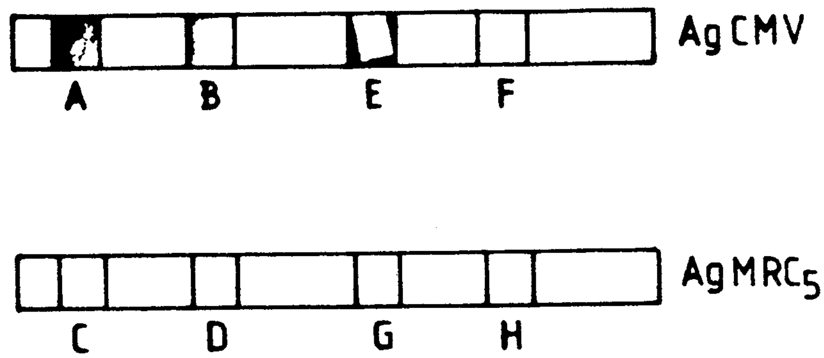


FIG.4

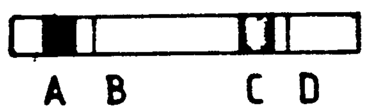


FIG.5

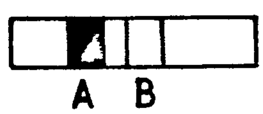


FIG.6

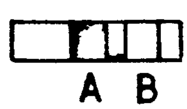


FIG.7

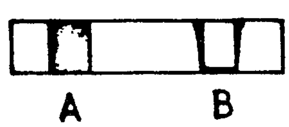

Office européen des brevets

# RAPPORT DE RECHERCHE EUROPEENNE

Numéro de la demande

EP 86 40 2611

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | WO-A-8 301 308 (MAST MEDICAL INDUSTRIES LTD) * Page 3, lignes 16-36; pages 4,5; revendications; figure 1; page 7 * | 1-7 | G 01 N 33/543<br>G 01 N 33/569 |
| A | | 11,16,<br>33,34 | |
| | --- | | |
| D,Y | FR-A-2 331 567 (INSERM) * Revendications * | 1-7 | |
| X | | 17,20,<br>21,34 | |
| | --- | | DOMAINES TECHNIQUES RECHERCHES (Int. Cl.4) |
| P,X | EP-A-0 163 799 (D. KÖHLER) * En entier; figure * | 1-3,13<br>-15,26 | G 01 N |
| | --- | | |
| X | EP-A-0 063 064 (LABORATOIRE DE STALLERGENES) * Pages 2,3; revendications 1,2,5 * | 26-28 | |
| | --- | | |
| X | EP-A-0 088 695 (CYTOGEN CORP.) * Revendications 1,2,7,22-24,33-34 * | 7-8,19<br>-22 | |
| | --- -/- | | |

Le présent rapport de recherche a été établi pour toutes les revendications

| Lieu de la recherche | Date d'achèvement de la recherche | Examinateur |
|---|---|---|
| LA HAYE | 24-02-1987 | MEYLAERTS H. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82

0230166

Office européen des brevets

# RAPPORT DE RECHERCHE EUROPEENNE

Numero de la demande

EP 86 40 2611

Page 2

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int Cl.4) |
|---|---|---|---|
| A | DE-A-2 733 380 (BEHRING-WERKE AG)<br>* Revendications 1-7 *<br>--- | 4,6-7 | |
| A | EP-A-0 152 886 (MOLECULAR DIAGNOSTICS)<br>--- | | |
| A | EP-A-0 122 841 (INSTITUT PASTEUR, INSERM)<br>--- | | |
| P,A | WO-A-8 505 638 (M. WILCHEK et al.)<br>----- | | |

DOMAINES TECHNIQUES RECHERCHES (Int. Cl.4)

Le présent rapport de recherche a été établi pour toutes les revendications

| Lieu de la recherche | Date d'achèvement de la recherche | Examinateur |
|---|---|---|
| LA HAYE | 24-02-1987 | MEYLAERTS H. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82